# EUROPEAN PATENT SPECIFICATION

(11) **EP 2 754 455 B1**
(45) Date of publication and mention of the grant of the patent: **31.08.2016**
(21) Application number: 12830532.3
(22) Date of filing: 04.09.2012
(51) Int. Cl.: A61M 1/00

(54) **NASAL ASPIRATION SYRINGE**
NASALE ASPIRATIONSSPRITZE
SERINGUE D'ASPIRATION NASALE

(30) Priority: 05.09.2011 ES 201100816
(43) Date of publication of application: 16.07.2014
(73) Proprietor: Ribelles Lopez, Angel, 08008 Barcelona (ES); Martinez Calderon, Blanca, 08008 Barcelona (ES)
(72) Inventor: RIBELLES LOPEZ, Angel, 08008 Barcelona (ES)
(74) Representative: Espiell Volart, Eduardo Maria
(86) International application number: PCT/ES2012/000229
(87) International publication number: WO 2013/034785

(56) References cited:
- WO-A1-2010/018288
- FR-A1- 2 535 611
- FR-A1- 2 564 321
- FR-A1- 2 919 808
- FR-A3- 2 632 528
- JP-A- 2003 052 807
- US-A1- 2009 118 680

## Description

### Object of the invention

The present invention refers to a nasal aspirator syringe, specifically designed to remove mucus in babies, without the need for a direct contact between the baby and the person who makes the removal of the mucus.

The object of the invention is to perform a controlled removal of mucus in babies, as well as to avoid aggressive stress that may hurt the baby itself and even avoid spreading microbes in a direct way, in the absence of any direct contact between the person who is doing the removal of mucus and the baby.

### BACKGROUND OF THE INVENTION

As is well known, the operation to carry out the removal of mucus in babies, is a difficult task, so that regardless of the means used to carry out the removal, there is always a direct contact between the baby and the person who performs the operation, and it may hurt the baby because he normally moves in front of the element that is inserted into his nose to remove the mucus.

On the other hand, the removal of mucus as is currently done is neither effective, nor, of course, controlled.

In addition, due to the direct contact between the person who is doing the removal and the baby, there is the possibility of microbes directly spreading.

Finally, also comment on the fact that, at no time, the amount of mucus extracted can be checked, even in an approximate way.

Document FR2564321 A1 discloses a device which can be manipulated using one hand and which allows suction of nasal mucous. The invention comprises the cylinder in which the plunger is displaced under the effect of the expansion of the spring which is kept primed by the catch which is manipulated by means of the pin situated at the front of the apparatus, to which is joined a cannula comprising a chamber which receives the mucous sucked out which cannot penetrate the body of the suction instrument, being stopped by the pad of fibre contained in the pocket, which is air-permeable and contains an antiseptic and bactericidal material, it being possible for the said canula to be disposable.

Document US 2009/118680 A1 discloses an improved consumable for use in a fluid/gas exchange in vitreoretinal surgery. The consumable includes a syringe having a barrel with a first opening for receiving a retinal tamponading gas, a stopper slidably disposed within and fluidly sealed to the barrel, a plug assembly, and a plunger. The stopper has a relief vlave for venting air and retinal tamponading gas to atmosphere via a hollow bore in the plunger.

### DESCRIPTION OF THE INVENTION

The nasal aspirator syringe that is being recommended has been designed to solve the above mentioned problems, on the basis of a simple but highly efficient solution.

More specifically, the syringe of the invention, being formed by a hollow body and an inner movable plunger, has the particularity of having, on the front end of the body, a cannula that has a conical end section, tightly coupled under pressure, with a configuration suitable for introducing it in the nose of the baby without hurting him, cannula that has a hole on its end, for the passage of the mucus that is to be removed from the nose of the baby.

In the interior of said cannula, a foam block, that is held against some longitudinal stops placed for that purpose in the interior of the cannula, is coupled, stops that are materialized by nerves placed in the direction of the generatrix.

Said cannula outwardly shows an annular projection as a stop of maximum penetration thereof inside the body of the syringe.

In turn, the plunger has a head with a rhombic profile but with very rounded vertexes, so that the rhombic head has a back extension determining a cylindrical neck that joins a trunk-conical section pertaining to the front part of the plunger itself, and whose trunk-conical section has slots for the passage of air through the plunger itself, during its introduction in the body of the syringe in an axial way.

On the cylindrical neck established between the rhombic head of the plunger and the trunk-conical section thereof, there is a joint with a perimeter lip of adjustment on the inner surface of the body of the syringe, and whose joint has a stop of maximum penetration in the body of the syringe upon this having an internal circumferential wall through which it is possible to move the head of the plunger but not the joint.

According to these structural characteristics, the suction will be carried out by backwardly retracting the plunger, carrying out the removal of mucus in the baby, the vacuum being established by the airtight fit that the joint performs on the lateral surface of the body of the syringe, also allowing, once the plunger has retracted, to return to the operating position by its moving forward, so that the air, as mentioned above, will exit through the openings or slots of the trunk-conical section of said plunger.

The backward movement of the plunger with respect to the body of the syringe, is limited by an inner annular ring corresponding to the rearest section of the body of the syringe.

In this way, mucus in babies is removed in a controlled way and aggressive stress that can hurt the baby is avoided.

Besides, there will be no contact between the baby and the person that carries out the removal at no time, since the only thing that needs to be done is to introduce the cannula in the nose of the baby and carry out the suction and corresponding removal of mucus, so that in the absence of any direct contact, there is no risk of spreading microbes directly.

As for the characteristics of the materials of which the above-mentioned components are made, mention that the cannula will be preferably transparent and made of a suitable material, such as PP random with a mixture of a thermoplastic elastomer.

In turn, the foam block, which will act as a stop to prevent the mucus that has been removed from being transferred to the cannula, will be made of polyester, preferably white, as well as the plunger, while the body of the syringe will be translucent and of a material similar to the one of the cannula, without discarding any other suitable types.

### DESCRIPTION OF THE DRAWINGS

To complement the description that is being made below and in order to help better understand the characteristics of the invention, according to a preferred practical embodiment thereof, a set of drawings is attached as an integral part of said description, wherein the following is shown as way of illustration but not limited to:

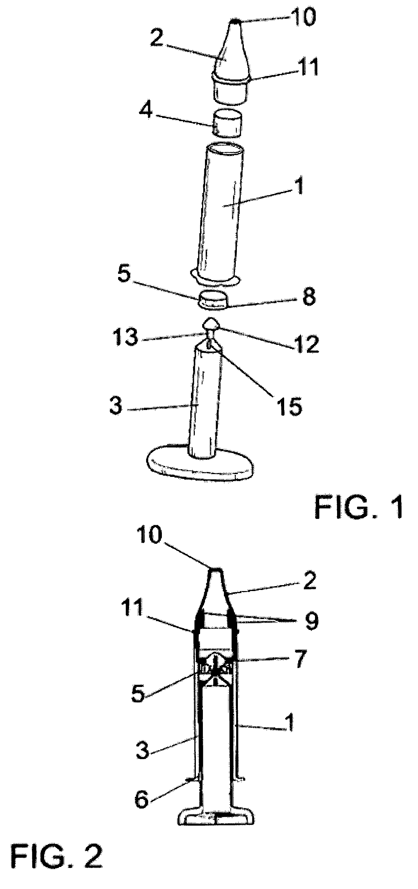

- Figure 1.-: shows a general perspective of the different components involved in the set-up of the nasal aspirator syringe object of the invention.
- Figure 2.-: shows a section view of the assemble and coupling among the different components represented in the figure above.
- Figure 3.-: shows a magnified detail and in section of the area or part corresponding to the syringe where the head of the plunger is placed along with the joint and the coupling end of the cannula on the body of the syringe.

### PREFERRED EMBODIMENT OF THE INVENTION

As shown in the figures above, the syringe of the invention comprises a hollow body (1), a cannula (2) and a plunge (3), as major components, and which are complemented with a foam block (4) and a joint (5) whose functionality will be discussed later.

The body (1) will be preferably translucent, having a ring (6) in the inner and lower part as a stop element of maximum extraction of the plunge (3) itself, while in proximity to the front end, said body (1) of the syringe includes a diametric wall or partition (7) by way of a nerve that acts as a stop which limits the joint (5) from moving forward, this having an annular nerve (8) on its base for an airtight fit on the wall or inner surface of the own wall of the body (1) of the syringe.

In turn, the foam block (4) is arranged in the interior of the cannula (2) and is held as a result of longitudinal nerves (9) provided in the inner face of the cannula (2) itself, thus preventing the foam block (4) from moving to the end (10) where the corresponding hole of the cannula (2) is placed, this preferably having a conical configuration on said end for its adaptation to the nostril of the baby in which it will be applied. Said cannula (2) also has an annular and external projection (11) as a stop limiting the maximum penetration on the body (1) of the syringe, since said cannula (2) couples on said body of the syringe under pressure, on the open upper end, i.e. the front, of said body (1).

The function of the foam block (4) is to hold the mucus extracted and avoid its passage to the remainder of components, constituting a disposable element, so that, once utilized, it should be substituted for a new one, keeping the body of the syringe with its plunger (3) and the joint (5) itself, which may be washed with water as usual.

The joint (5), with its lip (8) has the purpose of causing watertightness during the movement of the piston (3), in order to carry out the correct suctioning.

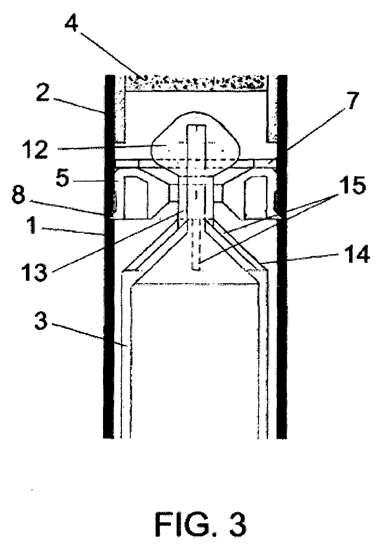

Finally, mention that the plunger (3) includes a head (12) with a rhombic configuration but with sharply rounded vertexes, whose head joins with the body of the plunger (3) through a cylindrical neck (13), and such joining is carried out on a trunk-conical section (14) that is part of the front end of the plunger (3) itself, as is clearly seen in figure 3, so that in correspondence with that trunk-conical section (14) some slots (15) for the exit of the air have been provided.

Functioning during the suctioning step and corresponding removal of the mucus, is as follows:
Placing the plunger forward on the body (1) of the syringe, and after the introduction of the cannula (2) in the nostril of the baby, the retraction and backward movement of the piston (3) will take place, so that through its rhombic head (12) it drags the joint (5), causing the joint (5) to be airtight and causing the corresponding suction of the mucus.

Once the limit of maximum backward movement of the plunger (3) with respect to the plunger (1) of the syringe has been reached, a movement forward can be performed, in order to carry out a second mucus removal operation, without the need to remove the cannula (2) from the nostril. Once the initial position of the plunger (3) has been reached, logically, the joint (5) will meet the inner annular wall or partition (7) and will establish the watertightness through its lip (8) against the inner surface of the body (1) of the syringe, allowing the air to exit through the slots (15) provided in the trunk-conical section (14) of the front of the plunger (3) itself, so that that air exits through those slots (15), through the plunger (3), as the density of the foam block (4) will offer the necessary resistance for the air to evaporate outward through those drillings or slots (15).

## Claims

1. Nasal aspirator syringe, which being preferably and fundamentally designed to carry out the removal of mucus in babies, avoiding direct contact between the person carrying out the removal of mucus and the baby itself, and being formed by a hollow and cylindrical body (1) open on both ends, and an axially movable plunger (3) inside said cylindrical body (1), comprising on the open front end of the cylindrical body (1) of the syringe, a detachable and disposable cannula (2) coupled under pressure, with an end hole for the passage of mucus that is suctioned from the nose of the baby by the backward movement of the corresponding plunger (3) along the cylindrical body (1) of the syringe; with the particularity that, inside said cannula (2), a foam block is placed, is **characterized in that** on the back of an inner diametric partition (7) of the body (1) of the syringe, a watertight joint (5) is mounted, located in a neck (13) or cylindrical section belonging to the plunger (3) itself, cylindrical section that is established between the end head (12) thereof, with a rhombic configuration with sharp rounded vertexes, and a trunk-conical section (14) as the front of the plunger (3) itself, being this trunk-conical section (14) provided with slots (15) for the exit and evaporation of the air during the forward movement phase of the plunger (3) itself.

2. Nasal aspirator syringe, according to claim 1, **characterized in that** the cannula (2) has a trunk-conical front section (14) and an appropriate configuration for its introduction and adaptation to the inside of the nostril of the baby, the body (1) of the cannula (2) having nerves (8) inside that work as stops of maximum movement toward the inside of the foam block itself housed inside said cannula (2), this also externally having, an annular projection that works as a stop of maximum penetration of the cannula (2) inside the cylindrical body (1) of the syringe.

3. Nasal aspirator syringe, according to claim 1, **characterized in that** the joint (5) mounted on the cylindrical section or neck (13) established between the rhombic head (12) and the front trunk-conical section (14) of the plunger (3), has a lower lip for watertightness and adjustment on the inner surface of the body (1) of the syringe, said joint (5) being movable forward and backwards and dragged in the corresponding movement of the plunger (3).

4. Nasal aspirator syringe, according to claims 1 and 3, **characterized in that** the back end (10) of the body (1) of the syringe has an inner ring establishing stop means, limiting the maximum removal of the plunger (3) during its backward movement inside said body (1) of the syringe.

## Patentansprüche

1. Nasensaugspritze, welche vorzugsweise und im Wesentlichen gestaltet ist, um das Entfernen von Schleim bei Kleinkindern durchzuführen, wobei ein direkter Kontakt zwischen der Person, die das Entfernen von Schleim durchführt, und dem Kleinkind selbst verhindert wird, und die durch einen hohlen und zylinderförmigen Körper (1), der an beiden Enden offen ist, und eine axial bewegliche Kolbenstange (3) innerhalb des zylinderförmigen Körpers (1) gebildet ist, die an dem offenen vorderen Ende des zylinderförmigen Körpers (1) der Spritze eine abnehmbare und austauschbare Kanüle (2) umfasst, die unter Druck gekoppelt ist, mit einem Endloch zum Durchgang von Schleim, der von der Nase des Kleinkinds durch die Rückwärtsbewegung der entsprechenden Kolbenstange (3) entlang des zylinderförmigen Körpers (1) der Spritze abgesaugt wird; mit der Besonderheit, dass innerhalb der Kanüle (2) ein Schaumblock platziert ist, **dadurch gekennzeichnet, dass** auf der Hinterseite einer inneren diametralen Trennwand (7) des Körpers (1) der Spritze ein wasserdichtes Anschlussstück (5) montiert ist, das in einem Hals (13) oder zylinderförmigen Abschnitt liegt, der zu der Kolbenstange (3) selbst gehört, wobei der zylinderförmige Abschnitt zwischen dem Endkopf (12) davon mit einer rautenförmigen Ausbildung mit scharfen abgerundeten Eckpunkten und einem kegelstumpfförmigen Abschnitt (14) an der Vorderseite der Kolbenstange (3) selbst eingerichtet ist, wobei dieser kegelstumpfförmige Abschnitt (14) mit Schlitzen (15) zum Ausströmen und Verdampfen der Luft während der Vorwärtsbewegungsphase der Kolbenstange (3) selbst versehen ist.

2. Nasensaugspritze nach Anspruch 1, **dadurch gekennzeichnet, dass** die Kanüle (2) einen kegelstumpfförmigen vorderen Abschnitt (14) und eine geeignete Ausbildung für ihr Einführen und ihre Anpassung in Bezug auf das Innere des Nasenlochs des Kleinkinds aufweist, wobei der Körper (1) der Kanüle (2) Rippen (8) im Inneren aufweist, die als Anschläge einer maximalen Bewegung zum Inneren des Schaumblocks selbst hin fungieren, der innerhalb der Kanüle (2) aufgenommen ist, wobei diese auch äußerlich einen ringförmigen Vorsprung aufweist, der als Anschlag für ein maximales Eindringen der Kanüle (2) innerhalb des zylinderförmigen Körpers (1) der Spritze fungiert.

3. Nasensaugspritze nach Anspruch 1, **dadurch gekennzeichnet, dass** das Anschlussstück (5), das auf dem zylinderförmigen Abschnitt oder Hals (13) montiert ist, der zwischen dem rautenförmigen Kopf (12) und dem vorderen kegelstumpfförmigen Abschnitt (14) der Kolbenstange (3) eingerichtet ist, eine untere Lippe für die Wasserdichtigkeit und Verstellung der inneren Fläche des Körpers (1) der Spritze aufweist, wobei das Anschlussstück (5) vorwärts und rückwärts bewegt werden kann und bei der entsprechenden Bewegung der Kolbenstange (3) mitgenommen wird.

4. Nasensaugspritze nach den Ansprüchen 1 bis 3, **dadurch gekennzeichnet, dass** das hintere Ende (10) des Körpers (1) der Spritze einen inneren Ring aufweist, der Anschlagmittel bildet, die das maximale Entfernen der Kolbenstange (3) während ihrer Rückwärtsbewegung innerhalb des Körpers (1) der Spritze einschränken.

## Revendications

1. Seringue-aspirateur nasale, qui est préférablement et essentiellement conçue pour effectuer l'élimination du mucus chez les bébés, en évitant un contact direct entre la personne qui effectue l'élimination du mucus et le propre bébé, et étant formée par un corps creux et cylindrique (1) ouvert sur les deux extrémités, et un piston amovible axialement (3) à l'intérieur dudit corps cylindrique (1), comprenant sur l'extrémité frontale ouverte du corps cylindrique (1) de la seringue, une canule détachable et jetable (2) couplée à pression, avec un trou d'extrémité pour le passage du mucus qui est aspiré du nez du bébé par le mouvement arrière du piston correspondant (3) le long du corps cylindrique (1) de la seringue ; ayant la particularité que, à l'intérieur de ladite canule (2), un bloc de mousse est placé, qui est **caractérisée en ce que à** l'arrière d'une paroi diamétrale interne (7) du corps (1) de la seringue, un joint d'étanchéité (5) est monté, localisé sur une section cylindrique ou col (13) appartenant au propre piston (3), section cylindrique qui est disposée entre sa tête d'extrémité (12), avec une configuration rhombique avec des sommets arrondis tranchants, et une section tronconique (14) à l'avant du propre piston (3), cette section tronconique (14) étant pourvue de fentes (15) pour l'évacuation et l'évaporation de l'air lors de la phase de mouvement avant du propre piston (3).

2. Seringue-aspirateur nasale, selon la revendication 1, **caractérisée en ce que** la canule (2) a une section frontale tronconique (14) et une configuration appropriée pour son introduction et adaptation à l'intérieur de la narine du bébé, le corps (1) de la canule (2) ayant des nerfs (8) à l'intérieur qui opèrent comme des butées du mouvement maximal vers l'intérieur du propre bloc de mousse logé à l'intérieur de ladite canule (2), ayant également à l'extérieur, une projection annulaire qui opère comme une butée de pénétration maximale de la canule (2) à l'intérieur du corps cylindrique (1) de la seringue.

3. Seringue-aspirateur nasale, selon la revendication 1, **caractérisée en ce que** le joint (5) monté sur le col (13) ou section cylindrique disposé entre la tête rhombique (12) et la section frontale tronconique (14) du piston (3), a une lèvre inférieure pour l'étanchéité et l'ajustement sur la surface interne du corps (1) de la seringue, ledit joint (5) étant déplaçable vers l'avant et vers l'arrière et entraîné dans le mouvement correspondant du piston (3).

4. Seringue-aspirateur nasale, selon les revendications 1 et 3, **caractérisée en ce que** l'extrémité arrière (10) du corps (1) de la seringue a un anneau interne constituant des moyens de butée, limitant l'extraction maximale du piston (3) lors de son mouvement vers l'arrière à l'intérieur dudit corps (1) de la seringue.
